# EUROPÄISCHE PATENTSCHRIFT

(11) **EP 0 595 107 B1**
(45) Veröffentlichungstag und Bekanntmachung des Hinweises auf die Patenterteilung: **20.05.1998**
(21) Anmeldenummer: 93116511.2
(22) Anmeldetag: 12.10.1993
(51) Int. Cl.: A61B 5/117, H01L 27/08

(54) **Sensoranordnung zur Erfassung von Fingerabdrücken und Verfahren zu deren Herstellung**
Sensor arrangement for recording finger prints and process for their manufacturing
Arrangement de palpeurs pour enregistrer les empreintes et procédé pour leur fabrication

(30) Priorität: 26.10.1992 DE 4236133
(43) Veröffentlichungstag der Anmeldung: 04.05.1994
(73) Patentinhaber: SIEMENS AKTIENGESELLSCHAFT, 80333 München (DE)
(72) Erfinder: Scheiter, Thomas, Dipl.-Ing., D-87751 Heimertingen (DE); Biebl, Markus, Dipl.-Phys., D-81543 München (DE); Klose, Helmut, Dr., D-81929 München (DE)

(56) Entgegenhaltungen:
- EP-A- 0 041 693
- EP-A- 0 044 489
- EP-A- 0 071 269
- APPLIED PHYSICS LETTERS, Bd.58, Nr.1, 7. Januar 1991, NEW YORK, US Seiten 100 - 102 T.W.KENNY ET AL 'micromachined silicon tunnel sensor for motion detection'
- PATENT ABSTRACTS OF JAPAN vol. 7, no. 282 (P-243) (1427) 16. Dezember 1983 & JP-A-58 158 778 (NIPPON DENSHIN DENWA KOSHA) 21. September 1983

## Beschreibung

Zur Erleichterung und der Automatisierung des Vergleichs von Fingerabdrücken ist eine Digitalisierung eines Fingerabdrucks und anschließende Speicherung erforderlich.

Es ist bekannt, nach Einfärbung der Fingerkuppe einen Positivabdruck der Linienstruktur des Fingerabdrucks zu erzeugen. Anschließend wird der Fingerabdruck mit Hilfe bekannter Bilderfassung, z. B. einer CCD-Kamera oder einem Scanner, digitalisiert.

Aus der europäischen Patentanmeldung 0 041 693 ist ein kapazitiver Sensor zur Erfassung von Fingerabdrücken bekannt, bei dem auf einer Einfärbung des Fingers verzichtet werden kann. Der Sensor umfaßt eine Vielzahl von kleinen Kondensatoren. Wird der Finger gegen eine Abtastoberfläche gepreßt, so verändern sich die Kapazitäten der Kondensatoren örtlich in Abhängigkeit des Fingerabdrucks. Die Kondensatoren sind als bewegliche Elektroden, die in einem flexiblen Isolator eingebettet sind oder als flexible Elektroden auf einem flexiblen Isolator, auf dessen gegenüberliegender Seite ortsfeste Einzelelektroden angeordnet sind, ausgebildet. Da der flexible Isolator als durchgehender Film realisiert ist, sind die einzelnen Kondensatoren miteinander mechanisch gekoppelt.

Ein weiterer Fingerabdrucksensor, der ohne Einfärbung des Fingers funktioniert, ist aus der europäischen Patentanmeldung 0 071 269 bekannt. Die Sensoranordnung umfaßt ein Feld oder eine Matrix von Transistoren, die neben der Kontaktfläche angeordnet sind. Die Operations-, Betriebs- und Arbeitspunkte dieser Transistoren werden direkt oder indirekt durch das Temperatur- oder Druckmuster des Fingerabdrucks beeinflußt. Dazu ist die Sensoranordnung an der Kontaktfläche mit einer piezoelektrischen oder pyroelektrischen durchgehenden Kristallschicht versehen. Für eine direkte Messung des Drucks höherer Temperatur können die Transistoren druck- oder temperaturabhängige Arbeitspunkte haben oder sie können jeweils in einen Schaltkreis integriert sein, der einen druck- oder temperaturabhängigen Widerstand aufweist.

Der Erfindung liegt das Problem zugrunde, eine Sensoranordnung anzugeben, mit der eine Erfassung und Speicherung von Fingerabdrücken ohne Einfärbung des Fingers möglich ist und mit der eine verbesserte Auflösung erzielt wird. Die Sensoranordnung soll insbesondere unempfindlich für Umgebungstemperaturschwankungen sein. Ferner soll ein Herstellverfahren für eine solche Sensoranordnung angegeben werden.

Dieses Problem wird erfindungsgemäß gelöst durch eine Sensoranordnung nach Anspruch 1. Die Sensoranordnung umfaßt ein Substrat aus Silizium, an dessen Oberfläche rasterförmig Membranen aus Polysilizium angeordnet sind, die gegenüber dem Substrat elektrisch isoliert sind. Jede Membran überspannt dabei einen Hohlraum und ist an mindestens zwei Stützstellen mit dem Substrat fest verbunden. Dabei ist der Hohlraum jeweils zwischen der Membran und der Oberfläche des Substrats angeordnet. Sowohl die Oberfläche des Substrats als auch die Membran sind dotiert und dadurch elekrisch leitfähig. Jede Membran definiert ein Sensorelement. Bei Einwirkung eines Fingers auf die Oberfläche zur Erfassung von Fingerabdrücken kommt es im Bereich der Linien der Fingerkuppe zu einer Berührung. In den Senken zwischen den Linien der Fingerkuppe findet keine Berührung der Membran statt. Es werden daher bei Einwirkung einer Fingerkuppe nur diejenigen Membranen aus der Ruhelage ausgelenkt, auf die infolge der Berührung der Linien eine Kraft einwirkt. Der Zustand der Membranen ist daher ein Abbild der Linienstruktur der Fingerkuppe.

Die Isolation zwischen den Membranen und dem Substrat wird jeweils durch pn-Übergänge oder durch Isolationsstrukturen, die im Bereich der Stützstellen zwischen dem Substrat und der jeweiligen Membran angeordnet sind, realisiert.

Durch Auslesen einer elektrischen Größe, die sich bei einer Auslenkung der Membran infolge Einwirkung einer Kraft auf die Membran ändert, wird daher ein digitales Bild des Fingerabdrucks registriert. Die Sensoranordnung umfaßt dazu eine Ausleseeinheit, die die entsprechende elektrische Größe ausliest und speichert.

Als elekrische Größe zur Beurteilung des Zustands jeder Membran ist z. B. das Potential zwischen der Membran und der Substratoberfläche oder eine Kapazitätsänderung zwischen der Membran und der Substratoberfläche geeignet.

Soll als elektrische Größe das Potential zwischen der Membran und der Substratoberfläche registriert werden, so ist die an den entsprechenden Hohlraum angrenzende Oberfläche des Substrats unbedeckt, so daß es bei Berührung der Membran mit der Substratoberfläche infolge einer Krafteinwirkung auf die Membran zu einem elektrischen Kontakt zwischen beiden kommt.

Soll dagegen die Kapazitätsänderung zwischen Membran und Substratoberfläche als Meßgröße verwendet werden, so wird zwischen der Membran und der Substratoberfläche ein Dielektrikum angeordnet. Dazu ist es vorteilhaft, an der Oberfläche des Substrats eine isolierende Schicht anzuordnen. Diese Anordnung hat darüberhinaus den Vorteil, daß Membranen, die nach Beendigung der Krafteinwirkung nicht in ihre Ruhelage zurückkommen und die deshalb bei nachfolgenden Aufnahmen das Ergebnis verfälschen würden, durch Anlegen eines entsprechenden elektrischen Potentials in die Ruhelage zurückbewegt werden können (Resetfunktion).

Um eine ausreichende Auflösung bei der Erfassung von Fingerabdrücken zu gewährleisten, liegt es im Rahmen der Erfindung, die Membranen mit rechteckigem Querschnitt vorzusehen, wobei Länge und Breite der Membran jeweils im Bereich zwischen 10 und 50 µm liegen.

Da die Sensorelemente der Sensoranordnung in einem Substrat aus Silizium realisiert sind, liegt es im Rahmen der Erfindung, die Ausleseeinheit mit der Sensoranordnung integriert in dem Substrat vorzusehen. Dieses ist wegen der Große der Sensorelemente vorteilhaft, da die geringen Abmessungen der einzelnen Sensorelemente eine herkömmliche Verdrahtung schwierig machen. Insbesondere über ein integriertes Schieberegister, mit denen die Membranen über Leiterbahnen, wie sie aus hochintegrierten Schaltungen der Mikroelektronik (VLSI-Technologien) bekannt sind, verbunden sind, sind die elektrischen Größen, die der Zustandsinformation jedes Sensorelementes entsprechen, vorteilhaft auszulesen.

Zur Erhöhung der Empfindlichkeit der Sensoranordnung liegt es im Rahmen der Erfindug, jede Membran mit einer Struktur zur Definition des Druckpunktes zu versehen. Als Struktur zur Definition des Druckpunktes ist z. B. eine Erhöhung mit rechteckigem Querschnitt aus Polysilizium geeignet, die in der Mitte der Membran angeordnet ist.

Um eine Beschädigung der Membranen bei einer Bewegung des Fingers parallel zur Oberfläche der Sensoranordnung zu vermeiden, liegt es im Rahmen der Erfindung, jede Membran mit einer Versteifung zur Begrenzung seitlicher Bewegungen zu versehen.

Zum Schutz der Oberfläche der Membran ist es vorteilhaft, die Oberfläche mit einer planarisierenden Schicht zu versehen, die die Membranen vollständig bedeckt. Dabei wird die Gefahr der Zerstörung der Membranen durch seitliche Bewegungen reduziert. Außerdem werden Beeinträchtigungen der Funktionsfähigkeit durch Schmutz, der durch die Berührung mit der Fingerkuppe nicht zu verhindern ist, vermieden. Ferner wird die Reinigung der Sensoranordnung erleichtert.

Das der Erfindung zugrundeliegende Problem wird weiterhin gelöst durch ein Verfahren zur Herstellung einer Sensoranordnung zur Erfassung von Fingerabdrücken gemäß Anspruch 12. Dabei wird auf die Oberfläche eines Substrats aus Silizium eine Si₃N₄-Schicht aufgebracht. Auf der Si₃N₄-Schicht wird eine SiO₂-Struktur erzeugt. Die SiO₂-Struktur umfaßt rasterförmig angeordnete SiO₂-Strukturelemente. Die einzelnen SiO₂-Strukturelemente sind z. B. quaderförmig. Darauf wird eine Polysiliziumstruktur erzeugt. Die Polysiliziumstruktur umfaßt zu jedem SiO₂-Strukturelement ein Polysiliziumstrukturelement. Das Polysiliziumstrukturelement überdeckt dabei das jeweilige SiO₂-Strukturelement vollständig und überragt es an mindestens zwei gegenüberliegenden Seiten seitlich. Die Polysiliziumstrukturelemente sind z. B. rechteckförmig. Anschließend wird die SiO₂-Struktur in einem Ätzprozeß, der selektiv zu Polysilizium und zu Si₃N₄ SiO₂ entfernt, entfernt. Dabei entstehen zwischen der Si₃N₄-Schicht und der Polysiliziumstruktur Hohlräume, die jeweils von einer Membran überspannt werden, die aus dem entsprechenden Teil der Polysiliziumstruktur gebildet wird. Das Herstellverfahren umfaßt aus der Mikroelektronik bekannte Strukturierungsschritte. Diese Art Herstellverfahren werden auch als mikromechanisch und, da die Strukturen an der Oberfläche hergestellt werden, als Surface-Micromachining bezeichnet.

Mit Hilfe des erfindungsgemäßen Herstellverfahrens ist es möglich, eine Sensoranordnung mit einer Auflösung im Bereich von ca. 20 µm zu erzielen. Eine Sensoranordnung zur Erfassung von Fingerabdrücken sollte wegen der entsprechenden Größe der Finger eine Gesamtfläche von etwa 2 cm² aufweisen. Bei einer Kantenlänge quadratischer Membranen der einzelnen Sensorelemente von 20 µm umfaßt die Sensoranordnung insgesamt 500.000 Sensorelemente. Eine solche Anordnung ist durch Einsatz der Surface-Micromachiningtechnologie gut herstellbar.

Zur Verbesserung des Ätzangriffs bei der Entfernung der SiO₂-Struktur liegt es im Rahmen der Erfindung, unter der Polysiliziumstruktur eine Hilfsstruktur, die selektiv zu Polysilizium ätzbar ist, zu erzeugen. Die Hilfsstruktur verlauft teilweise unterhalb der Polysiliziumstruktur außerhalb der SiO₂-Struktur und reicht bis in die Zwischenräume, die weder von der SiO₂-Struktur noch von der Polysiliziumstruktur bedeckt sind, hinein. Die Hilfsstruktur umfaßt z. B. Stege, die vom Rand eines SiO₂-Strukturelementes zum Rand des entsprechenden Polysiliziumstrukturelementes verlaufen. Auf diese Weise entstehen beim Ätzen Ätzkanäle, über die der Ätzangriff zugleich an mehreren Stellen erfolgt. Dadurch kann die Dauer für das Entfernen der SiO₂-Struktur und damit die Dauer des Ätzangriffs an nicht mit SiO₂ bedeckten Oberflächen der Si₃N₄-Schicht reduziert werden.

Ferner ermöglicht die Verwendung der Hilfsstruktur den Einsatz von Polysiliziumstrukturelementen, die das entsprechende SiO₂-Strukturelement jeweils an allen Seiten überlappen. Dadurch sind die Polysiliziumstrukturelemente entlang einer geschlossenen Ankerfläche, die das jeweilige SiO₂-Strukturelement rahmenförmig umgibt, mit der Oberfläche der Si₃N₄-Schicht oder der Hilfsstruktur verbunden. Jedes SiO₂-Strukturelement ist in dieser Ausführungsform vollständig durch das Polysiliziumstrukturelement abgeschlossen. In einem zu Polysilizium selektiven Ätzprozeß wird die Hilfsstruktur entfernt, wobei Ätzkanäle für die Entfernung der SiO₂-Strukturelemente gebildet werden. Die Ätzkanäle verlaufen entsprechend der Form der Hilfsstruktur unter dem Rand der Polysilizumstrukturelemente bis zu dem SiO₂-Strukturelement.

Es liegt im Rahmen der Erfindung, die Hilfsstruktur aus SiO₂ oder aus einer Kombination von SiO₂ und Si₃N₄ zu bilden.

Die Ätzkanäle können nach Entfernung der SiO₂-Strukturelemente in einem Oxidationsschritt wieder verschlossen werden.

Weitere Ausgestaltungen der Erfindung gehen aus den übrigen Ansprüchen hervor.

Im folgenden wird die Erfindung anhand der Figuren und der Ausführungsbeispiele näher erläutert.

Fig. 1 zeigt einen Ausschnitt einer Sensoranordnung zur Erfassung von Fingerabdrücken, die mit Strukturen zur Definition des Druckpunktes versehen ist.

Fig. 2 zeigt einen Ausschnitt einer Sensoranordnung zur Erfassung von Fingerabdrücken, die mit einer planarisierenden Schicht versehen ist.

Fig. 3 zeigt eine Draufsicht auf einen Ausschnitt einer Sensoranordnung zur Erfassung von Fingerabdrücken.

Fig. 4 zeigt einen Schichtaufbau, der zur Herstellung einer Sensoranordnung zur Erfassung von Fingerabdrücken erforderlich ist.

Fig. 5 zeigt den in Fig. 4 mit V-V bezeichneten Schnitt durch den Schichtaufbau.

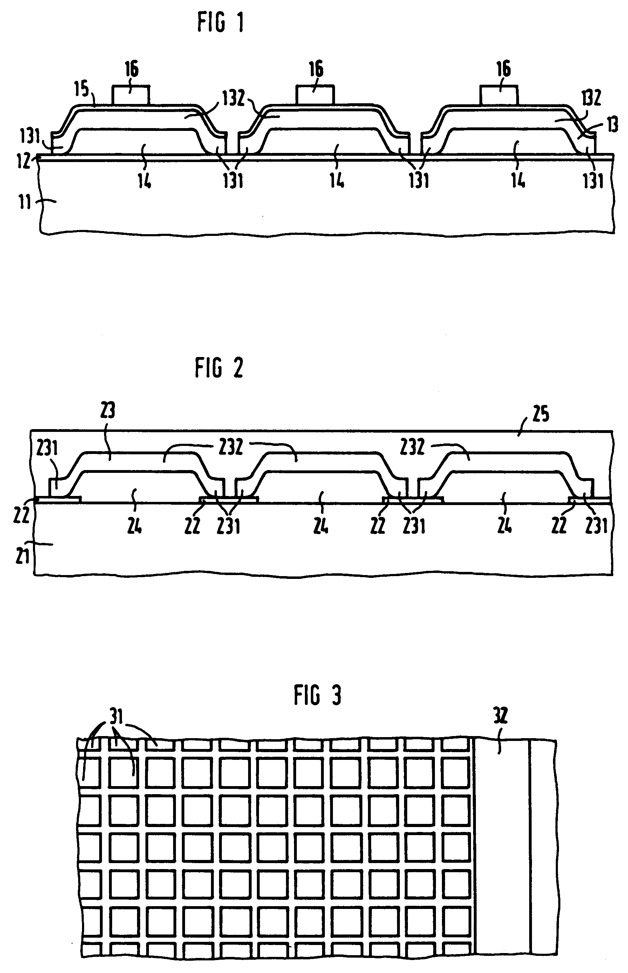

Auf einem Substrat 11 aus z. B. n-dotiertem Silizium ist eine isolierende Schicht 12 aus z. B. Si₃N₄ angeordnet (s. Fig. 1).

An der Oberfläche der isolierenden Schicht 12 ist eine Polysiliziumstruktur 13 angeordnet, die z. B. n-dotiert ist. Die Polysiliziumstruktur 13 umfaßt eine Vielzahl rasterförmig angeordneter Polysiliziumstrukturelemente, die z. B. rechteckförmig sind. Jedes Strukturelement der Polysiliziumstruktur 13 steht nur an Stützstellen 131 direkt mit der isolierenden Schicht 12 in Verbindung. Dazwischenliegende Teile des jeweiligen Strukturelementes der Polysiliziumstruktur 13 bilden Membrane 132, die jeweils einen Hohlraum 14 überspannen. Die Stützstellen 131 umgeben die zugehörige Membran 131 rahmenförmig, so daß die Membran 132 über eine geschlossene Ankerfläche, die von den Stützstellen 131 gebildet wird, umgeben ist. Der Hohlraum 14 ist abgeschlossen und wird durch die Oberfläche der isolierenden Schicht 12, die ihn umgebenden Stützstellen 131 und die zugehörige Membran 132 begrenzt. Die Länge der Membrane 132 beträgt z. B. 10 bis 50 µm. Die Dicke der Polysiliziumstruktur 13 senkrecht zur Oberfläche des Substrats 11 beträgt z. B. 0,8 µm.

Die Oberflache der Polysiliziumstruktur 13 ist mit einer passivierenden Schicht 15 aus z. B. Si₃N₄ bedeckt.

Oberhalb jeder Membran 132 ist eine Struktur zur Definition des Druckpunktes 16 angeordnet. Die Struktur 16 besteht z. B. aus Polysilizium, das als Erhebung mit rechteckigem Querschnitt ungefähr über der Mitte jeder Membran 132 angeordnet ist.

In der Sensoranordnung wird als elektrische Größe der Kapazitätsänderung zwischen jeder Membran 132 und dem Substrat 11 ausgewertet. Als Dielektrikum wirkt dabei derjenige Teil der isolierenden Schicht 12, der jeweils an einen Hohlraum 14 angrenzt. Da es für die Erfassung von Fingerabdrücken lediglich erforderlich ist, festzustellen, ob eine der Membranen 132 aus der Ruhelage ausgelenkt ist oder nicht, ist es bei der Registrierung der Kapazitätsänderung ausreichend, das Überschreiten der gemessenen Kapazität über eine vorgegebene Schwelle zu registrieren. Die Schwelle liegt zwischen dem Wert für Membran in der Ruhelage und dem Wert für ausgelenkte Membran. Für diese Messung ist daher eine Meßgenauigkeit im Bereich 100 fF ausreichend.

An der Oberfläche eines Substrats 21, das z. B. aus n-dotiertem Silizium besteht, ist eine Isolationsstruktur 22 aus z. B. Si₃N₄ angeordnet (s. Fig. 2). Die Isolationsstruktur 22 weist senkrecht zur Oberfläche des Substrats 21 eine Dicke von z. B. 50 nm auf.

Es ist eine Polysiliziumstruktur 23 vorgesehen, die z. B. n-dotiert ist. Die Polysiliziumstruktur 23 umfaßt eine Vielzahl rasterförmig angeordnete Strukturelemente. Die Strukturelemente der Polysiliziumstruktur 23 sind z. B. rechteckförmig. Die Strukturelemente der Polysiliziumstruktur 23 sind jeweils an Stützstellen 231 mit der Isolationsstuktur 22 fest verbunden. Der Anteil jedes Strukturelementes der Polysiliziumstruktur 23 zwischen benachbarten Stützstellen 231 überspannt dabei als Membran 232 einen Hohlraum 24. In dem Hohlraum 24 ist die Oberfläche des Substrats 21 mindestens teilweise freigelegt. Der Hohlraum 24 ist vollständig von Stützstellen 231 eines Strukturelementes der Polysiliziumstruktur 23 umgeben. Die Stützstellen 231 eines Strukturelementes der Polysiliziumstruktur 23 bilden eine geschlossene Ankerfläche, die den jeweiligen Hohlraum 24 vollständig rahmenförmig umgibt.

Die Polysiliziumstruktur 23 weist eine Dicke von z. B. 0,8 µm auf. Jede Membran 232 weist eine Seitenlänge von z. B. 10 bis 50 µm auf.

Die Oberfläche der Struktur ist mit einer planarisierenden Schicht 25 bedeckt. Die planarisierende Schicht 25 besteht z. B. aus BPSG. Sie schützt die Oberfläche der Polysiliziumstruktur 23 vor Zerstörung bei seitlichen Bewegungen und Verschmutzung und dient gleichzeitig zur Passivierung der Oberfläche.

Figur 3 zeigt eine Draufsicht auf einen Ausschnitt aus einer Sensoranordnung. Dabei sind Sensorelemente 31 rasterförmig an der Oberfläche eines Substrats angeordnet. Die Sensorelemente 31 umfassen jeweils eine Membran aus Polysilizium, die einen Hohlraum überspannt und die an mindestens zwei Stützstellen mit dem Substrat über jeweils eine Isolationsstruktur fest verbunden ist. Vorzugsweise ist die Membran entlang ihrem gesamten Rand mit der Isolationsstruktur fest verbunden. Seitlich der Sensorelemente 31 ist in dem Substrat eine Ausleseeinheit 32 angeordnet. Es sind elektrische Verbindungen zwischen der Ausleseeinheit und jeder Membran sowie dem Substrat vorgesehen. Die Ausleseeinheit 32 umfaßt z. B. ein Schieberegister, über das spaltenweise die Sensoranordnung ausgelesen wird.

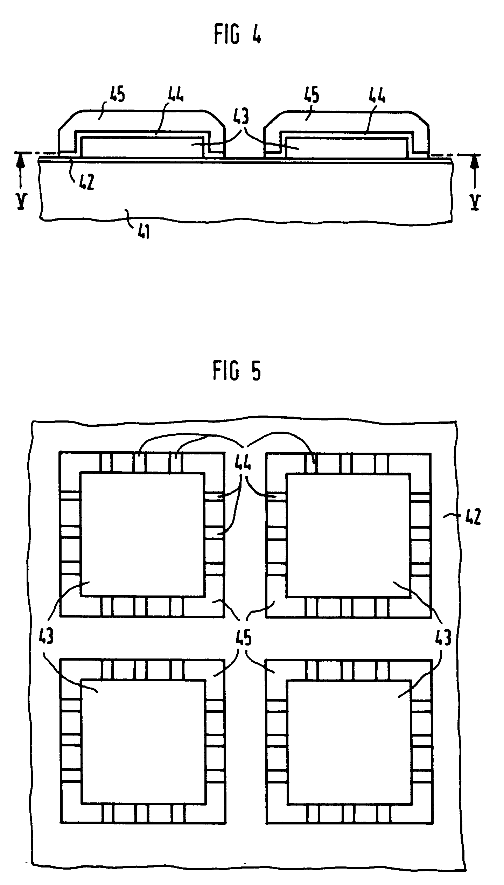

Anhand von Fig. 4 und 5 wird im folgenden ein Verfahren zur Herstellung einer Sensoranordnung zur Erfassung von Fingerabdrücken erläutert. An der Oberfläche eines Substrats 41, das z. B. aus n-dotiertem Silizium besteht, wird eine isolierende Schicht 42 aufgebracht, die z. B. aus Si₃N₄ besteht und die z. B. eine Dicke von 50 nm aufweist (s. Fig. 4). An der Oberfläche der isolierenden Schicht 42 wird durch ganzflächiges Abscheiden und anschließendes Strukturieren einer SiO₂₋Schicht eine SiO₂-Struktur 43 erzeugt. Die SiO₂-Struktur 43 umfaßt z. B. quader-förmige, rasterförmig angeordnete Strukturelemente. Die Strukturelemente der SiO₂-Struktur 43 haben eine Breite von z. B. 10 bis 50 µm. Die Dicke der SiO₂-Struktur 43 senkrecht zur Oberfläche des Substrats 41 beträgt z. B. 0,8 µm.

An der Oberfläche der SiO₂-Struktur sind Hilfsstrukturen 44 aus SiO₂ angeordnet. Die Hilfsstrukturen 44 überlappen die Strukturelemente der SiO₂-Struktur 43 seitlich. Die Hilfsstrukturen 44 weisen senkrecht zur Oberfläche des Substrats 41 eine Dicke von z. B. 50 nm auf.

Durch ganzflächiges Abscheiden und anschließendes Strukturieren einer Polysiliziumschicht wird eine Polysiliziumstruktur 45 erzeugt, die z. B. n-dotiert ist. Die Polysiliziumstruktur 45 umfaßt rechteckförmige, rasterförmig angeordnete Strukturelemente. Die Strukturelemente der Polysiliziumstruktur 45 überlappen die Strukturelemente der SiO₂-Struktur 43 vollständig. Der das Strukturelement der SiO₂-Struktur 43 seitlich überragende Teil des entsprechenden Strukturelementes der Polysiliziumstruktur 45 ist mit der isolierenden Schicht 42 bzw. der darauf angeordneten Hilfsstruktur 44 über eine Ankerfläche verbunden. Die Ankerfläche umgibt jedes Strukturelement der SiO₂-Struktur 43 in Form eines geschlossenen Rahmens.

In einem Ätzprozeß, z. B. naßchemisch unter Verwendung von Flußsäure, wird die Hilfsstruktur 44 aus z. B. SiO₂ selektiv zu Si₃N₄ und Polysilizium entfernt. Dabei entstehen Ätzkanäle, die unter der Ankerfläche jedes Strukturelementes der Polysiliziumstruktur 45 bis zu dem entsprechenden Strukturelement der SiO₂-Struktur 43 reichen. Über diese Ätzkanäle erfolgt bei Fortsetzung des Ätzprozesses ein Ätzangriff auf die SiO₂-Struktur 43. Die SiO₂-Struktur 43 wird dabei vollständig entfernt. Dabei entstehen Hohlräume, die jeweils von einem Teil eines Strukturelementes der Polysiliziumstruktur 45 überspannt werden. Die Hohlräume umgebend ist das Strukturelement der Polysiliziumstruktur 45 jeweils im Bereich der Ankerfläche über die isolierende Schicht 42 mit dem Substrat fest verbunden. Der einen Hohlraum überspannende Teil eines Strukturelementes der Polysiliziumstruktur 45 bildet eine Membran und definiert damit ein einzelnes Sensorelement der Sensoranordnung.

Da die Hilfsstrukturen 44 die Ankerflachen der Polysiliziumstruktur 45 seitlich überlappen, kommt es über die Hilfsstruktur 44 zu einem Ätzangriff auf die SiO₂-Struktur 43. Durch Entfernung der Hilfsstruktur 44 entstehende Kanäle außerhalb der Hohlräume werden bei nachfolgenden Prozeßschritten zur Fertigstellung der Sensoranordnung wieder verschlossen.

Soll der Zustand jeder Membran durch Messung des Potentials zwischen der Substratoberfläche und der Membran bestimmt werden, so muß die isolierende Schicht 42 vor der Erzeugung der SiO₂-Struktur 43 so strukturiert werden, daß die SiO₂-Struktur 43 mindestens teilweise unmittelbar an der Oberfläche des Substrats 41 angeordnet ist.

Zur Fertigstellung der Sensoranordnung wird z. B. ganzflächig eine planarisierende Schicht aufgebracht.

## Patentansprüche

1. Sensoranordnung zur Erfassung von Fingerabdrücken,
- bei der ein Substrat (11, 21) aus Silizium vorgesehen ist, das mindestens an der Oberfläche dotiert ist,
- bei der an der Oberfläche des Substrats (11, 21) rasterförmig Membranen (132, 232) aus dotiertem Polysilizium angeordnet sind, die gegenüber dem Substrat elektrisch isoliert sind,
- bei der jede Membran (132, 232) einen Hohlraum (14, 24) überspannt und an mindestens zwei Stützstellen (131, 231) mit dem Substrat (11, 21) festverbunden ist, so daß der Hohlraum (14, 24) zwischen der Membran (132, 232) und der Oberfläche des Substrats ()11, 21) angeordnet ist,
- bei der eine Ausleseeinheit vorgesehen ist, die eine elektrische Größe, jeweils zwischen Substrat (11, 21) und Membran (132, 232), die sich bei Einwirkung einer durch eine Linie einer Fingerkuppe bewirkten Kraft auf die Membran (132, 232) ändert, ausliest und speichert.

2. Sensoranordnung nach Anspruch 1,
- bei der die Membranen (132, 232) zeilenförmig angeordnet sind,
- bei der die Hohlräume (14, 24) jeweils entlang senkrecht zu den Zeilen verlaufenden Spalten angeordnet sind.

3. Sensoranordnung nach Anspruch 1 oder 2,
bei der mindestens im Bereich der Stützstellen (131, 231) zwischen der Membran (132, 232) und der Oberfläche des Substrats (11, 21) Isolationsstrukturen (12, 22) vorgesehen sind.

4. Sensoranordnung nach einem der Ansprüche 1 bis 3,
bei der die Oberfläche des Substrats mindestens im Bereich der Hohlräume mit einer isolierenden Schicht (12) versehen ist.

5. Sensoranordnung nach Anspruch 4,
bei der die Ausleseeinheit ein Schieberegister umfaßt, das die elektrische Größe spaltenweise ausliest und in einen Speicher einschreibt.

6. Sensoranordnung nach einem der Ansprüche 1 bis 5,
bei der jede Membran parallel zur Oberfläche des Substrats einen rechteckigen Querschnitt aufweist und die Länge und die Breite der Membran (132, 232) jeweils im Bereich zwischen 10 und 50 µm liegt.

7. Sensoranordnung nach einem der Ansprüche 1 bis 7,
bei der jede Membran (132) mit einer Struktur (16) zur Definition des Druckpunktes versehen ist.

8. Sensoranordnung nach einem der Ansprüche 1 bis 7,
bei der jede Membran (232) mit einer Versteifung zur Begrenzung seitlicher Bewegungen versehen ist.

9. Sensoranordnung nach einem der Ansprüche 1 bis 8,
bei der die Oberfläche mit einer planarisierenden Schicht (25) versehen ist, die die Membranen (232) vollständig bedeckt.

10. Sensoranordnung nach einem der Ansprüche 1 bis 9,
bei der die Hohlräume senkrecht zur Oberfläche des Substrats eine Höhe im Bereich zwischen 0,1 µm und 1 µm aufweisen.

11. Sensoranordnung nach einem der Ansprüche 1 bis 10,
bei der die Membranen (132, 232) in Richtung senkrecht zur Oberfläche des Substrats eine Dicke zwischen 0,2 µm und 2 µm aufweisen.

12. Verfahren zur Herstellung einer Sensoranordnung zur Erfassung von Fingerabdrücken,
- bei dem auf die Oberfläche eines Substrats (41) aus Silizium eine Si₃N₄-Schicht (42) aufgebracht wird,
- bei dem auf die Oberfläche der Si₃N₄-Schicht (42) eine SiO₂-Schicht aufgebracht wird,
- bei dem die SiO₂-Schicht strukturiert wird, so daß eine SiO₂-Struktur (43) entsteht, die rasterförmig angeordnete SiO₂-Strukturelemente umfaßt, und so daß die Oberfläche der Si₃N₄-Schicht (42) außerhalb der SiO₂-Struktur (43) freigelegt wird,
- bei dem ganzflächig eine Polysiliziumschicht abgeschieden und dotiert wird,
- bei dem die Polysiliziumschicht mit Hilfe eines zu SiO₂ und Si₃N₄ selektiven Ätzprozeß so strukturiert wird, daß eine Polysiliziumstruktur (45) entsteht, die zu jedem SiO₂-Strukturelement ein Polysiliziumstrukturelement umfaßt, wobei das Polysiliziumstrukturelement das SiO₂-Strukturelement vollständig überdeckt und an mindestens zwei gegenüberliegenden Seiten seitlich überragt,
- bei dem die SiO₂-Struktur (43) in einem Ätzprozeß, der selektiv zu Polysilizium und Si₃N₄ SiO₂ entfernt, entfernt wird, wobei zwischen der Si₃N₄-Schicht (42) und der Polysiliziumstruktur (45) Hohlräume entstehen, die jeweils von einer Membran, die aus dem entsprechenden Teil der Polysiliziumstruktur (45) gebildet wird, überspannt werden.

13. Verfahren nach Anspruch 12,
bei dem vor der Abscheidung der Polysiliziumschicht die Si₃N₄-Schicht (42) mindestens am Rand der Polysiliziumstrukturelemente entfernt wird.

14. Verfahren nach Anspruch 12 oder 13,
- bei dem die SiO₂-Schicht in einer Dicke im Bereich zwischen 0,1 µm und 1 µm abgeschieden wird,
- bei dem die Polysiliziumschicht in einer Dicke im Bereich zwischen 0,2 µm und 2 µm abgeschieden wird,
- bei dem die SiO₂-Strukturelemente (43) Ausdehnungen parallel zur Oberfläche im Bereich zwischen 10 und 50 µm aufweisen,
- bei dem die Polysiliziumstrukturelemente (45) Ausdehnungen parallel zur Oberfläche im Bereich zwischen 10 und 50 µm aufweisen.

15. Verfahren nach einem der Ansprüche 12 bis 14,
bei dem unter der Polysiliziumstruktur (45) eine Hilfsstruktur (44) erzeugt wird, die selektiv zu Polysilizium ätzbar ist und die Strukturelemente umfaßt, die mindestens vom Rand der SiO₂-Strukturelemente bis zum Rand der Polysiliziumstrukturelemente verlaufen und aus denen in einem zu Polysilizium selektiven Ätzprozeß Ätzkanäle für die Entfernung der SiO₂-Strukturelemente gebildet werden.

16. Verfahren nach Anspruch 15,
bei dem die Hilfsstruktur aus SiO₂ oder einer Kombination aus SiO₂ und Si₃N₄ gebildet wird.

17. Verfahren nach Anspruch 15 oder 16,
bei dem die Hilfsstruktur (44) eine Dicke senkrecht zur Oberflache des Substrats (41) im Bereich zwischen 20 nm und 200 nm aufweist.

18. Verfahren nach einem der Ansprüche 15 bis 17,
bei dem die Polysiliziumstrukturelemente die SiO₂-Strukturelemente jeweils an allen Seiten überlappen, so daß der Rand der Polysiliziumstrukturelemente entlang einer geschlossenen Ankerfläche, die das jeweilige SiO₂-Strukturelement rahmenförmig umgibt, mit der Oberfläche der Si₃N₄-Schicht oder der Hilfsstruktur verbunden ist.

19. Verfahren nach einem der Ansprüche 12 bis 18,
bei dem die Si₃N₄-Schicht (42) vor dem Aufbringen der SiO₂-Schicht so strukturiert wird, daß die Si₃N₄-Schicht (42) unter den Streifen der SiO₂-Schicht mindestens teilweise entfernt wird.

## Claims

1. Sensor arrangement for detecting fingerprints,
- in which a silicon substrate (11, 21) is provided which is doped at least on the surface,
- in which membranes (132, 232) of doped polysilicon are arranged in an array on the surface of the substrate (11, 21) and are electrically insulated from the substrate,
- in which each membrane (132, 232) spans a cavity (14, 24) and is firmly connected to the substrate (11, 21) at at least two support points (131, 231), so that the cavity (14, 24) is arranged between the membrane (132, 232) and the surface of the substrate (11, 21),
- in which a reading unit is provided which reads an electrical quantity, in each case between the substrate (11, 21) and the membrane (132, 232), which changes under the effect of a force on the membrane (132, 232) due to a line of a fingertip, and stores this quantity.

2. Sensor arrangement according to Claim 1,
- in which the membranes (132, 232) are arranged in rows,
- in which the cavities (14, 24) are each arranged along columns extending perpendicular to the rows.

3. Sensor arrangement according to Claim 1 or 2, in which insulation structures (12, 22) are provided at least in the region of the support points (131, 231) between the membrane (132, 232) and the surface of the substrate (11, 21).

4. Sensor arrangement according to one of Claims 1 to 3, in which the surface of the substrate is provided with an insulating layer (12) at least in the region of the cavities.

5. Sensor arrangement according to Claim 4, in which the reading unit comprises a shift register which reads the electrical quantity by columns and writes it into a memory.

6. Sensor arrangement according to one of Claims 1 to 5, in which each membrane has a rectangular cross-section parallel to the surface of the substrate, and the length and the width of the membrane (132, 232) are each in the region of between 10 and 50 µm.

7. Sensor arrangement according to one of Claims 1 to 7,
in which each membrane (132) is provided with a structure (16) for definition of the pressure point.

8. Sensor arrangement according to one of Claims 1 to 7,
in which each membrane (232) is provided with a stiffening for limiting lateral movements.

9. Sensor arrangement according to one of Claims 1 to 8,
in which the surface is provided with a planarizing layer (25) which fully covers the membranes (232).

10. Sensor arrangement according to one of Claims 1 to 9,
in which the cavities have, perpendicular to the surface of the substrate, a height in the region of between 0.1 µm and 1 µm.

11. Sensor arrangement according to one of Claims 1 to 10,
in which the membranes (132, 232) have, in the direction perpendicular to the surface of the substrate, a thickness of between 0.2 µm and 2 µm.

12. Process for producing a sensor arrangement for detecting fingerprints,
- in which an Si₃N₄ layer (42) is applied to the surface of a silicon substrate (41),
- in which an SiO₂ layer is applied to the surface of the Si₃N₄ layer (42),
- in which the SiO₂ layer is structured in such a way as to produce an SiO₂ structure (43) which comprises SiO₂ structural elements arranged in an array, and so that the surface of the Si₃N₄ layer (42) is exposed outside the SiO₂ structure (43),
- in which a polysilicon layer is deposited surface-wide and doped,
- in which the polysilicon layer is structured using an etching process which is selective with respect to SiO₂ and Si₃N₄, in such a way as to produce a polysilicon structure (45) which comprises a polysilicon structural element at each SiO₂ structural element, the polysilicon structural element fully covering the SiO₂ structural element and protruding laterally on at least two opposite sides,
- in which the SiO₂ structure (43) is removed in an etching process which removes SiO₂ selectively with respect to polysilicon and Si₃N₄, cavities being produced between the Si₃N₄ layer (42) and the polysilicon structure (45), each of which being spanned by a membrane which is formed from the corresponding part of the polysilicon structure (45).

13. Process according to Claim 12,
in which the Si₃N₄ layer (42) is removed, at least at the edge of the polysilicon structural elements, before the polysilicon layer is deposited.

14. Process according to Claim 12 or 13,
- in which the SiO₂ layer is deposited in a thickness in the region of between 0.1 µm and 1 µm,
- in which the polysilicon layer is deposited in a thickness in the region of between 0.2 µm and 2 µm,
- in which the SiO₂ structural elements (43) have dimensions, parallel to the surface, in the region of between 10 and 50 µm,
- in which the polysilicon structural elements (45) have dimensions, parallel to the surface, in the region of between 10 and 50 µm.

15. Process according to one of Claims 12 to 14, in which an auxiliary structure (44) is produced below the polysilicon structure (45), which is etchable the selectively with respect to polysilicon and comprises the structural elements which extend at least from the edge of the SiO₂ structural elements to the edge of the polysilicon structural elements and from which etching channels for the removal of the SiO₂ structural elements are formed in an etching process which is selective with respect to polysilicon.

16. Process according to Claim 15,
in which the auxiliary structure is formed from SiO₂ or a combination of SiO₂ and Si₃N₄.

17. Process according to Claim 15 or 16,
in which the auxiliary structure (44) has a thickness, perpendicular to the surface of the substrate (41), in the region of between 20 nm and 200 nm.

18. Process according to one of Claims 15 to 17,
- in which the polysilicon structural elements in each case overlap the SiO₂ structural elements on all sides, so that the edge of the polysilicon structural elements is connected to the surface of the Si₃N₄ layer or the auxiliary structure along a closed anchor face which frames the respective SiO₂ structural element.

19. Process according to one of Claims 12 to 18, in which the Si₃N₄ layer (42) is structured before the SiO₂ layer is applied, in such a way that the Si₃N₄ layer (42) is at least partly removed below the stripes of the SiO₂ layer.

## Revendications

1. Agencement de palpeurs pour l'enregistrement d'empreintes digitales,
- dans lequel on prévoit un substrat (11, 21) en silicium qui est dopé au moins sur la surface,
- dans lequel des membranes (132, 232) en polysilicium dopé sont disposées en forme de trame sur la surface du substrat (11, 21), les membranes étant isolées électriquement vis-à-vis du substrat,
- dans lequel chaque membrane (132, 232) surplombe un espace creux (14, 24) et est reliée solidement au substrat (11, 21) à au moins deux endroits de support (131, 231), si bien que l'espace creux (14, 24) est disposé entre la membrane (132, 232) et la surface du substrat (11, 21),
- dans lequel on prévoit une unité de lecture qui lit et mémorise une grandeur électrique, respectivement entre le substrat (11, 21) et la membrane (132, 232), qui se modifie lors de l'application d'une force exercée par une ligne d'un bout de doigt sur la membrane (132, 232).

2. Agencement de palpeurs selon la revendication 1,
- dans lequel les membranes (132, 232) sont disposées en lignes,
- dans lequel les espaces creux (14, 24) sont disposés respectivement le long de colonnes s'étendant perpendiculairement aux lignes.

3. Agencement de palpeurs selon la revendication 1 ou 2, dans lequel, au moins dans la zone des endroits de support (131, 231), on prévoit des structures d'isolation (12, 22) entre la membrane (132, 232) et la surface du substrat (11, 21).

4. Agencement de palpeurs selon l'une quelconque des revendications 1 à 3,
dans lequel la surface du substrat est munie d'une couche isolante (12) au moins dans la zone des espaces creux.

5. Agencement de palpeurs selon la revendication 4,
dans lequel l'unité de lecture comprend un registre à décalage qui lit la grandeur électrique par colonnes et l'inscrit dans une mémoire.

6. Agencement de palpeurs selon l'une quelconque des revendications 1 à 5,
dans lequel chaque membrane présente une section transversale rectangulaire parallèlement à la surface du substrat et dans lequel la longueur et la largeur de la membrane (132, 232) se situent respectivement dans le domaine entre 10 et 50 µm.

7. Agencement de palpeurs selon l'une quelconque des revendications 1 à 7, dans lequel chaque membrane (132) est munie d'une structure (16) pour la définition du point d'application.

8. Agencement de palpeurs selon l'une quelconque des revendications 1 à 7,
dans lequel chaque membrane (232) est munie d'un renforcement pour limiter les mouvements latéraux.

9. Agencement de palpeurs selon l'une quelconque des revendications 1 à 8,
dans lequel la surface est munie d'une couche ayant un effet d'aplanissement (25), la couche recouvrant complètement les membranes (232).

10. Agencement de palpeurs selon l'une quelconque des revendications 1 à 9,
dans lequel les espaces creux présentent, perpendiculairement à la surface du substrat, une hauteur dans le domaine entre 0,1 µm et 1 µm.

11. Agencement de palpeurs selon l'une quelconque des revendications 1 à 10,
dans lequel les membranes (132, 232) présentent, dans la direction s'étendant perpendiculairement à la surface du substrat, une épaisseur entre 0,2 µm et 2 µm.

12. Procédé pour la fabrication d'un agencement de palpeurs pour enregistrer des empreintes digitales,
- dans lequel on applique une couche (42) en Si₃N₄ sur la surface d'un substrat (41) en silicium,
- dans lequel on applique une couche en SiO₂ sur la surface de la couche (42) en Si₃N₄,
- dans lequel on structure la couche en SiO₂ de telle sorte que l'on obtienne une structure (43) en SiO₂ qui comprend des éléments de structure en SiO₂ disposés en forme de trame et de telle sorte que la surface de la couche (42) en Si₃N₄ est mise à nu en dehors de la structure (43) en SiO₂,
- dans lequel on dépose une couche en polysilicium recouvrant toute la surface et on la dope,
- dans lequel on structure la couche en polysilicium à l'aide d'un processus de gravure sélectif par rapport à SiO₂ et à Si₃N₄ de telle sorte que l'on obtienne une structure (45) en polysilicium qui comprend, par rapport à chaque élément de structure en SiO₂, un élément de structure en polysilicium, l'élément de structure en polysilicium recouvrant complètement l'élément de structure en SiO₂ et faisant saillie latéralement sur au moins deux côtés opposés,
- dans lequel on élimine la structure (43) en SiO₂ dans un processus de gravure qui élimine de manière sélective le SiO₂ par rapport au polysilicium et au Si₃N₄, des espaces creux apparaissant entre la couche (42) en Si₃N₄ et la structure (45) en polysilicium, ces espaces étant respectivement surplombés par une membrane qui est formée à partir de la partie correspondante de la structure (45) en polysilicium.

13. Procédé selon la revendication 12,
dans lequel, avant le dépôt de la couche en polysilicium, on élimine la couche (42) en Si₃N₄ au moins au bord des éléments de structure en polysilicium.

14. Procédé selon la revendication 12 ou 13,
- dans lequel la couche en SiO₂ est déposée avec une épaisseur dans le domaine entre 0,1 µm et 1 µm,
- dans lequel la couche en polysilicium est déposée avec une épaisseur dans le domaine entre 0,2 µm et 2 µm,
- dans lequel les éléments de structure (43) en SiO₂ présentent des dimensions parallèles à la surface dans le domaine entre 10 et 50 µm,
- dans lequel les éléments de structure (45) en polysilicium présentent des dimensions parallèles à la surface dans le domaine entre 10 et 50 µm.

15. Procédé selon l'une quelconque des revendications 12 à 14,
dans lequel on forme une structure auxiliaire (44) en dessous de la structure (45) en polysilicium, qui peut être gravée de manière sélective par rapport au polysilicium et qui comprend des éléments de structure qui s'étendent au moins depuis le bord des éléments de structure en SiO₂ jusqu'au bord des éléments de structure en polysilicium et à partir desquels, dans un processus de gravure sélectif par rapport au polysilicium, on forme des canaux de gravure pour l'élimination des éléments de structure en SiO₂.

16. Procédé selon la revendication 15,
dans lequel on forme la structure auxiliaire à partir de SiO₂ ou d'une combinaison de SiO₂ et de Si₃N₄.

17. Procédé selon la revendication 15 ou 16,
dans lequel la structure auxiliaire (44) représente une épaisseur perpendiculairement à la surface du substrat (41) dans le domaine entre 20 nm et 200 nm.

18. Procédé selon l'une quelconque des revendications 15 à 17,
dans lequel les éléments de structure en polysilicium chevauchent les éléments de structure en SiO₂, respectivement de tous côtés, si bien que le bord des éléments de structure en polysilicium est relié à la surface de la couche en Si₃N₄ ou de la structure auxiliaire, le long d'une surface d'ancrage fermée qui entoure à la manière d'un cadre l'élément de structure en SiO₂ respectif.

19. Procédé selon l'une quelconque des revendications 12 à 18,
dans lequel on structure la couche (42) en Si₃N₄ avant l'application de la couche en SiO₂ de telle sorte que la couche (42) en Si₃N₄ est éliminée au moins partiellement en dessous des bandes de la couche en SiO₂.
